(19) Europäisches Patentamt / European Patent Office / Office européen des brevets

(11) **EP 3 650 832 B1**

(12) **EUROPÄISCHE PATENTSCHRIFT**

(45) Veröffentlichungstag und Bekanntmachung des Hinweises auf die Patenterteilung:
**14.01.2026 Patentblatt 2026/03**

(21) Anmeldenummer: **18204943.7**

(22) Anmeldetag: **07.11.2018**

(51) Internationale Patentklassifikation (IPC):
*G01N 17/00* *(2006.01)*

(52) Gemeinsame Patentklassifikation (CPC):
**G01N 17/002; G01N 33/0083; H01M 8/04305;**
Y02E 60/50

(54) **PRÜFKAMMER FÜR BRENNSTOFFZELLEN UND VERFAHREN ZUR STEUERUNG**

TEST CHAMBER FOR FUEL CELLS AND METHOD FOR CONTROLLING

CHAMBRE DE TEST POUR DES PILES À COMBUSTIBLE ET PROCÉDÉ DE COMMANDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.05.2020 Patentblatt 2020/20**

(73) Patentinhaber: Weiss Technik GmbH
**35447 Reiskirchen (DE)**

(72) Erfinder: WAGNER, Enno
**55127 Mainz (DE)**

(74) Vertreter: advotec.
**Patent- und Rechtsanwaltspartnerschaft mbB Georg-Schlosser-Straße 6 35390 Gießen (DE)**

(56) Entgegenhaltungen:
DE-U1- 20 013 299

• L JÖRISSEN ET AL: "Fuel Cells for Stationary, Automotive and Portable Applications", 14 November 2001 (2001-11-14), XP055588754, Retrieved from the Internet <URL:https://www.digatron.com/fileadmin/pdf/bericht_fct.pdf> [retrieved on 20190515]

• DETLEF NAUNDORF ET AL: "Prüfsysteme für alternative Antriebe", 1 January 2005 (2005-01-01), XP055588734, Retrieved from the Internet <URL:https://link.springer.com/content/pdf/10.1007/BF03221725.pdf> [retrieved on 20190515]

• ZSW: "// Brennstoffzellen, Batterien und Superkondensatoren // Modellierung // Materialien // Komponenten // Systeme // Testzentrum", 1 January 2008 (2008-01-01), XP055588727, Retrieved from the Internet <URL:https://www.zsw-bw.de/fileadmin/user_upload/PDFs/Broschueren_und_Flyer/ZSW_Fachbroschuere_Ulm_dt.pdf> [retrieved on 20190515]

• ESPEC: "Temperature Chamber Series", 1 April 2018 (2018-04-01), pages 1 - 24, XP055908707, Retrieved from the Internet <URL:https://www.espec.co.jp/english/products/catalog/pvph.pdf> [retrieved on 20220404]

EP 3 650 832 B1

**Beschreibung**

Die Erfindung betrifft eine Prüfkammer, insbesondere Klimakammer zur Konditionierung von Luft, sowie ein Verfahren zur Steuerung einer Prüfkammer, wobei in einem Prüfraum der Prüfkammer eine Brennstoffzellenanordnung zumindest einer physikalischen Prüfbedingung ausgesetzt wird, wobei die Brennstoffzellenanordnung zumindest eine elektrochemische Brennstoffzelle mit einem Anodenraum und einem Kathodenraum mit jeweils einer Zuführöffnung zur Zuführung von Reaktanten und einer Abführöffnung zur Abführung von Abprodukten der Brennstoffzellenanordnung aufweist, wobei die Brennstoffzellenanordnung in dem Prüfraum betrieben wird, wobei der Brennstoffzellenanordnung als Reaktant ein Brenngas und ein im Prüfraum vorhandenes Oxidationsgas zugeführt wird, wobei die Prüfbedingung durch eine Steuerung und/oder Regelung einer Lufttemperatur, eines Luftdrucks und einer relativen Luftfeuchte in dem Prüfraum mittels einer Steuervorrichtung der Prüfkammer eingestellt wird, wobei mittels einer raumlufttechnischen Anlage der Prüfkammer dem Prüfraum konditionierte Zuluft zugeführt und aus dem Prüfraum Abluft abgeführt wird, wobei die raumlufttechnische Anlage von der Steuervorrichtung gesteuert wird.

Derartige Prüfkammern werden regelmäßig zur Überprüfung von physikalischen und/oder chemischen Eigenschaften von Gegenständen, insbesondere Vorrichtungen eingesetzt. So sind Temperaturprüfschränke oder Klimaprüfschränke bekannt, innerhalb derer Temperaturen in dem Bereich von -70 °C bis +180 °C eingestellt werden können. Bei Klimaprüfschränken können ergänzend gewünschte Klimabedingungen eingestellt werden, denen dann die Vorrichtung bzw. das Prüfgut über einen definierten Zeitraum ausgesetzt wird. Eine Temperierung eines das zu prüfende Prüfgut aufnehmenden Prüfraums erfolgt regelmäßig in einem Umluftkanal innerhalb des Prüfraums. Der Umluftkanal bildet einen Luftbehandlungsraum im Prüfraum aus, in dem Wärmetauscher zur Erwärmung oder Kühlung der den Umluftkanal bzw. den Prüfraum durchströmenden Luft angeordnet sind. Dabei saugt ein Lüfter bzw. ein Ventilator die im Prüfraum befindliche Luft an und leitet sie im Umluftkanal zu den jeweiligen Wärmetauschern. Das Prüfgut kann so temperiert oder auch einem definierten Temperaturwechsel ausgesetzt werden. Während eines Prüfintervalls kann dann beispielsweise eine Temperatur zwischen einem Temperaturmaximum und einem Temperaturminimum der Prüfkammer wechseln.

Weiter ist es bekannt, sogenannte Brennstoffzellen bzw. Brennstoffzellenanordnungen in einer derartigen Prüfkammer definierten Klimabedingungen auszusetzen, um beispielsweise eine Leistungsfähigkeit oder Funktionsfähigkeit der Brennstoffzellenanordnung zu überprüfen. Die aus dem Stand der Technik bekannten Brennstoffzellenanordnungen bzw. Brennstoffzellen sind mit einer Elektrolytmembran mit einer Anode und einer Kathode und beispielsweise Wasserstoff als Brenngas und Sauerstoff als Oxidationsgas bekannt.

Als Festpolymer-Brennstoffzellen sind sie regelmäßig aus einer polymerischen Ionenaustauschmembran mit einer beidseitigen, einen Elektrolytkatalysator ausbildenden Beschichtung aus einem porösen, elektrisch leitfähigen, die Anode bzw. die Kathode ausbildenden Schichtmaterial gebildet. Zwei elektrisch leitende Seperatorplatten decken die Elektrolytmembran beidseitig ab, wobei die Seperatorplatten Kanäle ausbilden, über die die Reaktanten in geeigneter Weise über die jeweilige Oberfläche der Elektrolytmembran verteilt werden.

Weiter dienen die Seperatorplatten als Stromkollektor im Bereich der Anode bzw. Kathode. Da eine mit einer herkömmlichen Brennstoffzelle erzielbare Ausgangsspannung vergleichsweise gering ist, werden zum Erreichen höherer Ausgangsspannungen Brennstoffzellen in Reihe geschaltet, das heißt in einer Stapelkonfiguration zu sogenannten Stacks angeordnet. Die Seperatorplatten sind dann als eine Bipolarplatte mit beidseitig ausgebildeten Kanälen zur Verteilung und Durchleitung der Reaktanten ausgebildet. Die anoden- bzw. kathodenseitigen Kanäle werden jeweils über eine einzelne Zuführöffnung mit Brenngas bzw. Oxidationsgas versorgt, wobei eine Abführung von Abprodukten über jeweils eine einzelne Abführöffnung erfolgt.

Wenn eine Brennstoffzellenanordnung mit Elektrolytmembranen mit Wasserstoff als Brenngas betrieben wird, sammelt sich während des Betriebs der Brennstoffzellenanordnung regelmäßig Prozesswasser und Kondensat im Anodenraum, der, um einer Flutung des Anodenraums vorzubeugen, regelmäßig gespült wird. Das im Anodenraum befindliche Wasser gelangt dann zusammen mit dem darin befindlichen Brenngas in eine Umgebung der Brennstoffzellenanordnung bzw. in den Prüfraum. Im Falle von Wasserstoff als Brenngas kann dies zur Bildung von Knallgas in dem Prüfraum führen, weshalb mittels der raumlufttechnischen Anlage ein Luftwechsel in dem Prüfraum erfolgen muss. Auch sind Brennstoffzellenanordnungen bekannt, bei denen Wasserstoff als Brenngas zirkuliert wird und angereicherte Fremdprodukte ausgeschieden werden müssen. Mittels der raumlufttechnischen Anlage wird im Prüfraum konditionierte Zuluft zugeführt und Abluft abgeführt. Dazu sind jeweils Kanäle für die Zuluft und die Abluft an den Prüfraum angeschlossen.

Zur Überprüfung der Brennstoffzellenanordnung wird diese mit einer elektronischen Last verbunden, wodurch über eine Erhöhung oder Verringerung eines elektrischen Widerstands Strom-Spannungs-Kennlinien erfasst werden können. Der Prüfraum und die darin angeschlossene raumlufttechnische Anlage sind dabei gegenüber einer Umgebungsluft des Prüfraums dicht abgeschlossen, wobei die Kathode der Brennstoffzellenanordnung mit Sauerstoff als Oxidationsgas aus dem Prüfraum versorgt wird.

Wesentlich für eine optimale Funktion der Brennstoffzellenanordnung innerhalb des Prüfraums ist die optimale Einstellung der relativen Luftfeuchte in dem Prüfraum. Wenn die in dem Prüfraum befindliche Luft der Brennstoffzellenanordnung mit Sauerstoff als Oxidationsgas zugeführt wird, darf diese nicht zu feucht oder zu trocken sein, da sonst die Leistungsdichte der Brennstoffzellenanordnung stark abnimmt. Auch muss die von der raumlufttechnischen Anlage zur Verfügung gestellte Zuluft hinsichtlich der Lufttemperatur entsprechend vorkonditioniert sein. Die nicht als Oxidationsgas nutzbaren Bestandteile der Luft, wie beispielsweise Stickstoff, müssen ebenfalls entsprechend konditioniert werden. Die raumlufttechnische Anlage muss daher über leistungsstarke Trockner und Befeuchter verfügen, wenn die für den erforderlichen Luftwechsel notwendige Luft entsprechend konditioniert werden soll. Weiter wird gegebenenfalls mittels der raumlufttechnischen Anlage ein Überdruck im Prüfraum ausgebildet, wodurch der Luftdurchsatz noch weiter erhöht wird.

Aus der DE 200 13 299 U1 ist eine Klimakammer bzw. Prüfkammer zum Prüfen von Brennstoffzellen bekannt. Dabei ist vorgesehen in einem Umluftkanal innerhalb eines Prüfraums der Prüfkammer über ein Ventil Pressluft als ein Spülgas einzuleiten. Die Pressluft bzw. das Spülgas wird im Umluftkanal vorkonditioniert und über einen Auslass aus dem Prüfraum wieder abgeführt. Weiter verfügt die Prüfkammer über Sensoren zur Detektion von im wesentlichen Brenngasen, wobei mittels eines Regel- und Überwachungssystems der Prüfkammer eine Brenngaskonzentration im Prüfraum überwacht und geregelt werden soll. Das Spülgas dient insbesondere dazu, eine Entstehung explosionsgefährlicher Gasgemische im Prüfraum zu verhindern.

Der vorliegenden Erfindung liegt daher die Aufgabe zugrunde, ein Verfahren sowie eine Prüfkammer mit einer Brennstoffzellenanordnung vorzuschlagen, mit dem bzw. der eine einfache und kostengünstige Prüfung einer Brennstoffzellenanordnung ermöglicht wird. Diese Aufgabe wird durch ein Verfahren mit den Merkmalen des Anspruchs 1 und eine Vorrichtung mit den Merkmalen des Anspruchs 18 gelöst.

Bei dem erfindungsgemäßen Verfahren zur Steuerung einer Prüfkammer, insbesondere einer Klimakammer zur Konditionierung von Luft, wird in einem Prüfraum der Prüfkammer eine Brennstoffzellenanordnung zumindest einer physikalischen Prüfbedingung ausgesetzt, wobei die Brennstoffzellenanordnung zumindest eine elektrochemische Brennstoffzelle mit einem Anodenraum und einem Kathodenraum mit jeweils einer Zuführöffnung zur Zuführung von Reaktanten und einer Abführöffnung zur Abführung von Abprodukten der Brennstoffzellenanordnung aufweist, wobei die Brennstoffzellenanordnung in dem Prüfraum betrieben wird, wobei der Brennstoffzellenanordnung als Reaktanten ein Brenngas und ein im Prüfraum vorhandenes Oxidationsgas zugeführt wird, wobei die Prüfbedingung durch eine Steuerung und/oder Regelung einer Lufttemperatur, eines Luftdrucks und einer relativen Luftfeuchte in dem Prüfraum mittels einer Steuervorrichtung der Prüfkammer eingestellt wird, wobei mittels einer raumlufttechnischen Anlage der Prüfkammer dem Prüfraum konditionierte Zuluft zugeführt und aus dem Prüfraum Abluft abgeführt wird, wobei die raumlufttechnische Anlage von der Steuervorrichtung gesteuert wird, wobei eine Sauerstoffkonzentration mittels eines Sensors einer Regeleinrichtung der Steuervorrichtung in dem Prüfraum bestimmt wird, wobei die Regeleinrichtung die für einen Betrieb der Brennstoffzellenanordnung erforderliche Sauerstoffkonzentration regelt.

Bei dem erfindungsgemäßen Verfahren wird demnach die Brennstoffzellenanordnung in dem Prüfraum der Prüfkammer angeordnet und betrieben. Dabei wird die Brennstoffzellenanordnung während eines Betriebs in einem Prüfzeitabschnitt definierten Umweltbedingungen ausgesetzt. In der raumlufttechnischen Anlage wird ein Luftwechsel in dem Prüfraum ausgebildet, um eventuell freigesetztes Brenngas der Brennstoffzellenanordnung aus dem Prüfraum zu entfernen. Insbesondere wird mit dem Sensor der Regeleinrichtung die Sauerstoffkonzentration in einer Luft des Prüfraums bestimmt bzw. gemessen und nach der für einen Betrieb der Brennstoffzellenanordnung erforderlichen Sauerstoffkonzentration in dem Prüfraum geregelt. Dadurch wird es möglich, eine Leistung der raumlufttechnischen Anlage so weit zu reduzieren, dass sich eine gerade ausreichende Sauerstoffkonzentration zum Betrieb der Brennstoffzellenanordnung in der Luft des Prüfraums einstellt. Es ist dann nicht mehr erforderlich, mit der raumlufttechnischen Anlage große Mengen an vorkonditionierter Zuluft dem Prüfraum zuzuführen, da nur so viel Luft bzw. Frischluft im Prüfraum eingeleitet werden muss, wie die Brennstoffzellenanordnung tatsächlich benötigt. Die Brennstoffzellenanordnung nutzt insbesondere den Sauerstoffanteil der Luft als Oxidationsgas, wobei die raumlufttechnische Anlage auch die übrigen Luftanteile wie Stickstoff entsprechend vorkonditionieren muss. Da mit dem erfindungsgemäßen Verfahren der von der raumlufttechnischen Anlage zu fördernde Sauerstoffanteil herabgesetzt werden kann, ist im Ergebnis eine vergleichsweise wesentlich kleinere Luftmenge von der raumlufttechnischen Anlage zu fördern und zu behandeln. Neben der so erzielbaren Einsparung an Energie ist es dann auch möglich, die raumlufttechnische Anlage und insbesondere die zur Behandlung der Zuluft erforderlichen Befeuchter, Entfeuchter und Wärmetauscher vergleichsweise kleiner zu dimensionieren, wodurch eine wesentliche Einsparung von Kosten erzielt werden kann.

Erfindungsgemäß wird die raumlufttechnische Anlage von der Steuervorrichtung gesteuert. Die Steuervorrichtung kann dabei von der Regeleinrichtung Daten oder Signale zur Sauerstoffkonzentration in dem Prüfraum übermittelt bekommen. Die Regeleinrichtung kann demnach zusammen mit der Steuervorrichtung eine Regelstrecke ausbilden. Die Steuervorrichtung kann dann beispielsweise die raumlufttechnische Anlage zur Vorkonditionierung der Zuluft und den Prüfraum zur Konditionierung der Luft in dem Prüfraum, insbesondere der Lufttemperatur und der relativen Luftfeuchte, regeln. Diese Regelung kann beispielsweise mittels eines PID-Reglers erfolgen. Die Regeleinrichtung der Steuervorrichtung kann zur Regelung der Sauerstoffkonzentration einen Luftwechsel in dem Prüfraum durch die Steuerung der raumlufttechnischen Anlage erhöhen oder vermindern.

Besonders vorteilhaft ist es, wenn in Abhängigkeit der Sauerstoffkonzentration mit einem Dosierventil der Regeleinrichtung Sauerstoff in den Prüfraum und/oder in einen Zuführkanal der raumlufttechnischen Anlage eingeleitet wird. Beispielsweise kann reiner Sauerstoff dann direkt in den Prüfraum über eine Zuführleitung eingeleitet werden. Der von der Brennstoffzellenanordnung verbrauchte Sauerstoff kann dann durch den in dem Prüfraum eingeleiteten Sauerstoff ersetzt werden. Es ist so nicht mehr erforderlich, den Sauerstoff über die Zuluft in den Prüfraum einzubringen, wodurch eine wesentlich geringere Menge an Zuluft von der raumlufttechnischen Anlage vorkonditioniert werden muss. Die raumlufttechnische Anlage kann dann noch kleiner dimensioniert werden. Alternativ oder ergänzend kann vorgesehen sein, in den Zuluftkanal der raumlufttechnischen Anlage reinen Sauerstoff einzuleiten, sodass die Zuluft einen erhöhten Anteil an Sauerstoff aufweist. Dieser Sauerstoff ist dann bereits vorkonditioniert. Der übrige Anteil der Luftbestandteile, der von der Brennstoffzellenanordnung nicht als Oxidationsgas nutzbar ist, ist dann entsprechend geringer. Insgesamt muss dann mit der raumlufttechnischen Anlage vergleichbar weniger konditionierte Zuluft dem Prüfraum zugeführt werden. Über das Dosierventil ist es möglich, die tatsächlich benötigte Menge an Sauerstoff relativ genau in den Prüfraum oder den Zuluftkanal zu dosieren, wodurch die zu konditionierende Luftmenge auf ein Minimum reduziert wird. Das Dosierventil kann beispielsweise ein elektronisches Regelventil sein.

Weiter kann vorgesehen sein, dass die Abprodukte in den Prüfraum abgegeben werden. Die Abprodukte können beispielsweise Wasser, Wasserdampf und Reste von Brenngas enthalten. Die Abprodukte können dann mit der Abluft der raumlufttechnischen Anlage aus dem Prüfraum abgeführt werden. Weiter können die Abprodukte bzw. der Wasserdampf zur Einstellung der Luftfeuchte im Prüfraum benutzt werden. Zumindest wird so sichergestellt, dass die Brennstoffzellenanordnung unter realistischen Umgebungsbedingungen geprüft wird.

Mittels einer Pumpe der Brennstoffzellenanordnung kann dem Kathodenraum in dem Prüfraum befindliche Luft zugeführt werden. Diese Luft weist dann die mittels des Sensors gemessene Sauerstoffkonzentration bzw. die mit der Regeleinrichtung geregelte Sauerstoffkonzentration in dem Prüfraum auf. Die Pumpe kann beispielsweise ein Kompressor sein, der ohnehin zum Betrieb der Brennstoffzellenanordnung erforderlich ist.

Mittels eines Brenngasdosierventils der Brennstoffzellenanordnung kann dem Anodenraum als ein Brenngas Wasserstoff zugeführt werden. So kann dann reiner Wasserstoff ($H_2$) über eine Zuführleitung direkt der Brennstoffzellenanordnung zugeleitet werden. Das Brenngasdosierventil kann ein elektronisches Regelventil sein. Mit der Reaktionsgleichung der Brennstoffzelle

$$H_2 + \frac{1}{2} O_2 \rightarrow H_2O + E_{el}$$

ergibt sich ein Verhältnis von Wasserstoff zu Sauerstoff von 2:1, wodurch die benötigte Menge an Sauerstoff mittels der Regeleinrichtung in Abhängigkeit der Wasserstoffmenge sehr genau in den Prüfraum eingeleitet werden kann. Eine zu konditionierende Luftmenge kann so noch weiter verringert werden.

Der Wasserstoff ($H_2$) und der Sauerstoff ($O_2$) können auch mittels eines in dem Prüfraum befindlichen Elektrolyseurs hergestellt werden. Beispielsweise kann in dem Prüfraum ein Druck-Elektrolyseur angeordnet sein, der die Menge an Wasserstoff und Sauerstoff produziert und in den Prüfraum abgibt, die von der Brennstoffzellenanordnung verbraucht wird. Damit kann auf eine aufwendige Installation von Wasserstoff- und Sauerstoffversorgungsleitungen mit der entsprechenden Bevorratung vollständig verzichtet werden. Es ist lediglich erforderlich, den Druck-Elektrolyseur mit elektrischer Energie innerhalb des Prüfraums zu versorgen. Prinzipiell ist es auch möglich, den Druck-Elektrolyseur außerhalb des Prüfraums anzuordnen und den Wasserstoff und den Sauerstoff über Versorgungsleitungen in die Brennstoffzellenanordnung bzw. in den Prüfraum über einen Zuluftkanal der raumlufttechnischen Anlage einzuleiten.

Die Lufttemperatur in dem Prüfraum kann mittels einer Temperiervorrichtung der Prüfkammer eingestellt werden. Somit wird es möglich, die vorkonditionierte Zuluft noch genauer zu temperieren. Die Regelung der Lufttemperaturen in dem Prüfraum erfolgt dann mittels der Steuervorrichtung. In dem Prüfraum kann dann auch ein Luftbehandlungsraum ausgebildet sein, in dem Wärmetauscher bzw. Wärmeübertrager zur Erwärmung oder Kühlung der den Prüfraum durchströmenden Luft angeordnet sind. Weiter kann ein Lüfter bzw. ein Ventilator zur Umsetzung der im Prüfraum befindlichen Luft vorgesehen sein, sodass der Luftbehandlungsraum in Art eines Umluftkanals ausgebildet ist.

Die relative Luftfeuchte in dem Prüfraum kann mittels eines in dem Prüfraum angeordneten Befeuchters und/oder eines Entfeuchters der Prüfkammer eingestellt werden. Der Befeuchter bzw. Entfeuchter kann in einem Luftbehandlungsraum in dem Prüfraum angeordnet sein. Die Steuervorrichtung kann über den Befeuchter bzw. Entfeuchter dann eine relative Luftfeuchte in dem Prüfraum relativ genau regeln. Gleichwohl kann die raumlufttechnische Anlage ebenfalls Befeuchter und/oder Entfeuchter zur Vorkonditionierung der Zuluft aufweisen.

Mittels eines Feuchtesensors eines Befeuchterregelkreises der Steuervorrichtung kann die relative Luftfeuchte in dem Prüfraum gemessen werden. So wird es möglich, eine relative Luftfeuchte in dem Prüfraum besonders genau zu regeln und einzustellen.

Der Luftdruck in dem Prüfraum kann mittels eines Zuluftgebläses und/oder eines Abluftgebläses der raumlufttechnischen Anlage eingestellt werden. Beispielsweise ist es dann so auch möglich, in dem Prüfraum einen Überdruck gegenüber einer Umgebung des Prüfraums auszubilden. Da in einem in sich geschlossenen Prüfraum durch die Zuführung von Brenngas und den Verbrauch von Oxidationsgas ein Luftdruck veränderlich wäre, kann so auch sichergestellt werden, dass stets ein konstanter Luftdruck in dem Prüfraum eingestellt werden kann. Eine Regelung des Zuluftgebläses bzw. des Abluftgebläses kann mittels der Steuervorrichtung erfolgen.

So kann mittels der raumlufttechnischen Anlage eine Luftwechselrate in dem Prüfraum und/oder eine Druckdifferenz zwischen dem Prüfraum und einer Umgebung ausgebildet werden. Die Luftwechselrate kann dabei so bemessen sein, dass sich stets eine nicht zündfähige Menge an Brenngas in dem Prüfraum befindet.

Ebenso kann die Lufttemperatur und/oder die relative Luftfeuchte der Zuluft mittels der raumlufttechnischen Anlage eingestellt werden. In diesem Fall weist die raumlufttechnische Anlage dann einen Befeuchter und/oder einen Entfeuchter sowie einen Wärmetauscher bzw. Wärmeübertrager zur Temperierung der Zuluft auf. Die Zuluft kann entsprechend der gewünschten Prüfbedingung für die Brennstoffzellenanordnung mittels der raumlufttechnischen Anlage vorkonditioniert werden. Eine genaue Einstellung der Prüfbedingung durch Konditionierung der im Prüfraum befindlichen Luft kann innerhalb des Prüfraums selbst erfolgen.

Besonders vorteilhaft ist es, wenn ein mit der Brennstoffzellenanordnung erzeugter elektrischer Strom gemessen werden kann, wobei die Steuervorrichtung die relative Luftfeuchte in Abhängigkeit des erzeugten Stroms regeln kann. Der gemessene elektrische Strom und eine mit der Brennstoffzellenanordnung erzeugte Menge an Wasser sind nach dem Faraday-Gesetz voneinander abhängig:

$$\dot{n}_{H2O,prod} = \frac{I}{n*F}$$

I=elektrischer Strom

n=2

F=96485 C/mol (Faradaykonstante)

Ein Stoffstrom des mit der Brennstoffzellenanordnung produzierten Wassers bzw. Wasserdampfs kann so unmittelbar und in Echtzeit mittels der Steuervorrichtung ermittelt werden. Auch kann die so gemessene, produzierte Wassermenge zur Vorsteuerung eines Feuchtereglers der Steuervorrichtung zur Einstellung der relativen Luftfeuchte in dem Prüfraum und/oder in der konditionierten Zuluft genutzt werden. Insgesamt ist es so möglich, die relative Luftfeuchte in dem Prüfraum und/oder der Zuluft in Abhängigkeit des erzeugten Stroms noch genauer zu regeln.

Alternativ oder ergänzend kann ein mit der Brennstoffzellenanordnung erzeugter elektrischer Strom gemessen werden, wobei die Regeleinrichtung die Sauerstoffkonzentration in Abhängigkeit des erzeugten Stroms regeln kann. Nach dem oben bezeichneten Faraday-Gesetz und der Reaktionsgleichung der Brennstoffzellenanordnung ist dann sehr genau bestimmbar, wie viel Sauerstoff zur Erzeugung des gemessenen elektrischen Stroms aus dem Prüfraum verbraucht wurde. Dieser Sauerstoff kann dann dem Prüfraum in der erforderlichen Menge entsprechend genau zugeführt werden. Die so bestimmte Menge an verbrauchtem Sauerstoff kann beispielsweise zur Vorsteuerung der Regeleinrichtung zur Regelung der Sauerstoffkonzentration eingesetzt werden.

Weiter kann der gemessene elektrische Strom als eine Eingangsgröße eines Führungsreglers einer Kaskadenregelung der Steuervorrichtung verwendet werden. Die Kaskadenregelung kann unter anderem einen PID-Regler zur Regelung der Lufttemperatur, des Luftdrucks und/oder der Luftfeuchte in dem Prüfraum umfassen. Eine von der Brennstoffzellenanordnung durch die Abgabe von Wasserdampf erhöhte relative Luftfeuchte als Steuergröße und ein Verbrauch an Sauerstoff kann von der Steuervorrichtung berücksichtigt werden, sodass eine Prüfung einer Brennstoffzellenanordnung mit einer Prüfkammer wesentlich vereinfacht wird. Eine zeitaufwendige Parametrisierung bzw. Voreinstellung dieser Variablen an der Steuervorrichtung der Prüfkammer ist dann nicht mehr erforderlich.

Besonders vorteilhaft ist es auch, wenn die Regeleinrichtung aus einem Folgeregler der Kaskadenregelung verwendet wird. So kann eine Regelgenauigkeit noch weiter verbessert werden.

Die physikalische Prüfbedingung kann eine Temperatur, eine relative Luftfeuchte, eine Korrosionsatmosphäre und/oder eine Bauteilfestigkeit sein. Weiter kann vorgesehen sein, die Brennstoffzellenanordnung in einem Prüfzeitabschnitt zu betreiben und unter Last dieser Prüfbedingung auszusetzen.

Die erfindungsgemäße Prüfkammer, insbesondere Klimakammer zur Konditionierung von Luft, umfasst einen gegenüber einer Umgebung verschließbaren und temperaturisolierten Prüfraum mit einer darin angeordneten Brennstoffzellenanordnung, die in dem Prüfraum betreibbar und zumindest einer klimatischen Prüfbedingung aussetzbar ist, wobei die Brennstoffzellenanordnung zumindest eine elektrochemische Brennstoffzelle mit einem Anodenraum und einem Kathodenraum mit jeweils einer Zuführöffnung zur Zuführung von Reaktanten und einer Abführöffnung zur Abführung von Abprodukten der Brennstoffzellenanordnung aufweist, wobei der Brennstoffzellenanordnung als Reaktanten ein Brenngas und ein im Prüfraum vorhandenes Oxidationsgas zuführbar ist, wobei die Prüfkammer eine Steuervorrichtung zur Einstellung der Prüfbedingung durch eine Steuerung und/oder Regelung einer Lufttemperatur, eines Luftdrucks und einer relativen Luftfeuchte in dem Prüfraum aufweist, wobei die Prüfkammer eine raumlufttechnische Anlage zur Zuführung konditionierter Zuluft in den Prüfraum und zur Abführung von Abluft aus dem Prüfraum aufweist, wobei die raumlufttechnische Anlage von der Steuervorrichtung steuerbar ist, wobei die Steuervorrichtung eine Regeleinrichtung mit einem Sensor zur Bestimmung einer Sauerstoffkonzentration in dem Prüfraum aufweist, wobei die Regeleinrichtung zur Regelung der für einen Betrieb der Brennstoffzellenanordnung erforderlichen Sauerstoffkonzentration in den Prüfraum ausgebildet ist. Zu den vorteilhaften Wirkungen der erfindungsgemäßen Prüfkammer wird auf die Vorteilsbeschreibung des erfindungsgemäßen Verfahrens verwiesen.

Die Prüfkammer kann eine Temperiervorrichtung zur Temperierung des Prüfraums aufweisen, wobei mittels der Temperiervorrichtung eine Temperatur in einem Temperaturbereich von -70 °C bis +180 °C, vorzugsweise -80 °C bis +200 °C innerhalb des Prüfraums ausbildbar sein kann, wobei die Temperiervorrichtung eine Kühleinrichtung mit einem Kühlkreislauf mit einem Kältemittel, einem Wärmeübertrager, der in dem Prüfraum angeordnet ist, einem Verdichter, einem Kondensator und einem Expansionsorgan aufweisen kann, wobei die Temperiervorrichtung eine Heizeinrichtung mit einer Heizung und einem weiteren Wärmeübertrager aufweisen kann.

Dann wird es möglich, eine umgewälzte Luftmenge des Prüfraums mittels der Kühleinrichtung über den Wärmeübertrager im Prüfraum abzukühlen. Der Wärmeübertrager kann wiederum an den Kühlkreislauf angeschlossen bzw. in diesem integriert sein, sodass das im Kühlkreislauf zirkulierende Kältemittel durch den Wärmeübertrager strömt. Der Verdichter kann beispielsweise ein Kompressor sein und dem Verdichter nachfolgend, und in Strömungsrichtung des Kältemittels kann der Kondensator für das verdichtete Kältemittel angeordnet sein. Das im Kondensator verflüssigte Kältemittel kann weiter über das Expansionsorgan strömen, sodass es durch Expansion in Folge eines Druckabfalls wiederum gasförmig wird und den Wärmeübertrager kühlt. Die Heizeinrichtung bzw. die Heizung kann aus elektrischen Heizelementen ausgebildet sein, über die der weitere Wärmeübertrager beheizbar ist. Der weitere Wärmeübertrager kann wie der Wärmeübertrager innerhalb des Prüfraums angeordnet sein.

Weitere vorteilhafte Ausführungsformen der Prüfkammer ergeben sich aus den Merkmalsbeschreibungen der auf den Verfahrensanspruch 1 zurückbezogenen Unteransprüche.

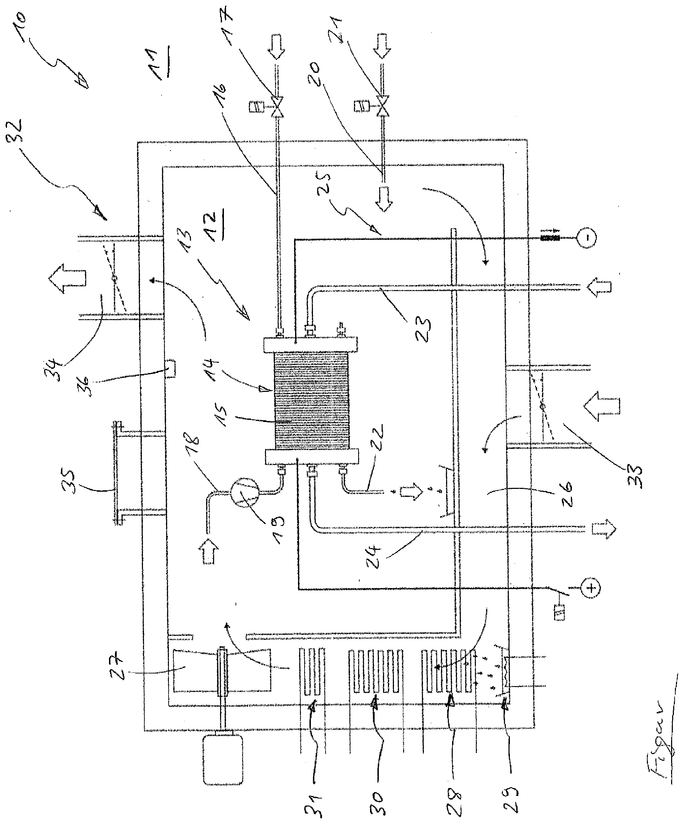

Im Folgenden wird die Erfindung unter Bezugnahme auf die beigefügte Zeichnung näher erläutert.

Die **Figur** zeigt eine schematische Darstellung einer Prüfkammer 10 mit einem gegenüber einer Umgebung 11 dicht abgeschlossenen Prüfraum 12, der temperaturisoliert ist. In dem Prüfraum 12 ist eine Brennstoffzellenanordnung 13 angeordnet, wobei die Brennstoffzellenanordnung 13 aus einem Stapel 14 aus Brennstoffzellen 15 ausgebildet ist. Der Brennstoffzellenanordnung 13 ist über eine Zuführleitung 16 mit einem Brenngasdosierventil 17 Wasserstoff ($H_2$) als ein Brenngas direkt zuführbar. Weiter ist der Brennstoffzellenanordnung 13 über eine Zuführleitung 18 mit einem Kompressor 19 in dem Prüfraum 12 befindliche Luft zuführbar. Darüber hinaus kann über eine Zuführleitung 20 mit einem Dosierventil 21 Sauerstoff ($O_2$) als ein Oxidationsgas eingeleitet werden. Abprodukte der Brennstoffzellenanordnung 13, wie Brenngasreste, Wasser und/oder Wasserdampf, werden über eine Abführleitung 22 in den Prüfraum 12 abgegeben.

Die Brennstoffzellenanordnung 13 wird mittels Kühlwasser gekühlt, wobei das Kühlwasser über eine Vorlaufleitung 23 der Brennstoffzellenanordnung 13 zugeführt wird und nach einem Durchströmen des Stapels 14 über eine Rücklaufleitung 24 wieder abgeführt wird. Die Brennstoffzellenanordnung 13 ist an einen Stromkreis 25 angeschlossen, über den eine Last erzeugt und eine Messung von Strom und Spannung erfolgen kann.

Innerhalb des Prüfraums 12 ist ein Umluftkanal 26 ausgebildet, durch den mittels eines Lüfters 27 im Prüfraum 12 befindliche Luft zirkuliert werden kann. In dem Umluftkanal 26 ist ein Entfeuchter 28, ein Befeuchter 29, ein Wärmeübertrager 30 zur Kühlung und ein weiterer Wärmeübertrager 31 zur Erwärmung der Luft angeordnet. Eine hier nur teilweise dargestellte raumlufttechnische Anlage 32 ist mit einem Zuluftkanal 33 und einem Abluftkanal 34 an den Prüfraum 12 angeschlossen. Über den Zuluftkanal 33 kann vorkonditionierte Zuluft unmittelbar in den Umluftkanal 26 und damit in den Prüfraum 12 eingeleitet werden. Eine Ausleitung der im Prüfraum 12 befindlichen Luft erfolgt über den Abluftkanal 34. Weiter ist an dem Prüfraum 12 eine Berstscheibe 35 angeschlossen. In dem Prüfraum 12 ist darüber hinaus ein Sensor 36 zur Messung einer Sauerstoffkonzentration in dem Prüfraum 12 angeordnet. Der Sensor 36 ist Teil einer hier nicht dargestellten Regeleinrichtung zur Regelung der Sauerstoffkonzentration. Die Regeleinrichtung steuert das Dosierventil 21 derart, dass die Sauerstoffkonzentration innerhalb des Prüfraums 12 stets durch eine Zuführung von Sauerstoff über die Zuführleitung 20 so eingestellt wird, dass die Brennstoffzellenanordnung 13 betrieben werden kann. So ist es möglich, den von der Brennstoffzellenanordnung 13 verbrauchten Sauerstoff der Luft im Prüfraum 12 über die Zuführleitung 20 nachzudosieren, ohne dass große Mengen an vorkonditionierter Luft über den Zuluftkanal 33 eingeleitet werden müssten.

Die Regeleinrichtung ist Teil einer hier nicht dargestellten Steuervorrichtung der Prüfkammer 10, wobei die Steuervorrichtung die raumlufttechnische Anlage 32, den Entlüfter 27, den Entfeuchter 28, den Befeuchter 29, den Wärmeübertrager 30 und den weiteren Wärmeübertrager 31 zur Konditionierung der im Prüfraum 12 befindlichen Luft regelt. Durch die Messung des von der Brennstoffzellenanordnung 13 erzeugten Stroms ist es weiterhin möglich, dass die Steuervorrichtung eine von der Brennstoffzellenanordnung 13 erzeugte Menge an Abprodukten bzw. Wasser und/oder an verbrauchtem Sauerstoff berechnet. Die Steuervorrichtung kann dann die raumlufttechnische Anlage 32, den Entfeuchter 28 und den Befeuchter 29 sowie das Dosierventil 21 entsprechend genau regeln bzw. steuern. Insgesamt muss dann die raumlufttechnische Anlage 32 nur noch eine geringe Menge Luft umwälzen, und eine Befeuchtung bzw. Entfeuchtung von Luft kann im Wesentlichen auf den Sauerstoffanteil der im Prüfraum 12 befindlichen Luft beschränkt werden. Darüber hinaus ist es möglich, auf eine aufwendige Parametrisierung der Steuervorrichtung vor der Durchführung eines Prüfablaufs mit der Brennstoffzellenanordnung 13 zu verzichten, da die betreffenden Variablen von der Steuervorrichtung selbst ermittelt werden können.

**Patentansprüche**

1. Verfahren zur Steuerung einer Prüfkammer (10), insbesondere einer Klimakammer zur Konditionierung von Luft, wobei in einem Prüfraum (12) der Prüfkammer eine Brennstoffzellenanordnung (13) zumindest einer physikalischen Prüfbedingung ausgesetzt wird, wobei die Brennstoffzellenanordnung zumindest eine elektrochemische Brennstoffzelle (15) mit einem Anodenraum und einem Kathodenraum mit jeweils einer Zuführöffnung zur Zuführung von Reaktanten und einer Abführöffnung zur Abführung von Abprodukten der Brennstoffzellenanordnung aufweist, wobei die Brennstoffzellenanordnung in dem Prüfraum betrieben wird, wobei der Brennstoffzellenanordnung als Reaktanten ein Brenngas und ein im Prüfraum vorhandenes Oxidationsgas zugeführt wird, wobei die Prüfbedingung durch eine Steuerung und/oder Regelung einer Lufttemperatur, eines Luftdrucks und einer relativen Luftfeuchte in dem Prüfraum mittels einer Steuervorrichtung der Prüfkammer eingestellt wird, wobei mittels einer raumlufttechnischen Anlage (32) der Prüfkammer dem Prüfraum konditionierte Zuluft zugeführt und aus dem Prüfraum Abluft abgeführt wird, wobei die raumlufttechnische Anlage von der Steuervorrichtung gesteuert wird, **dadurch gekennzeichnet,** **dass** eine Sauerstoffkonzentration mittels eines Sensors (36) einer Regeleinrichtung der Steuervorrichtung in dem Prüfraum bestimmt wird, wobei die Regeleinrichtung die für einen Betrieb der Brennstoffzellenanordnung erforderliche Sauerstoffkonzentration regelt.

2. Verfahren nach Anspruch 1, **dadurch gekennzeichnet,** **dass** in Abhängigkeit der Sauerstoffkonzentration mit einem Dosierventil (21) der Regeleinrichtung Sauerstoff ($O_2$) in den Prüfraum (12) und/oder in einen Zuluftkanal (33) der raumlufttechnischen Anlage (32) eingeleitet wird.

3. Verfahren nach Anspruch 1 oder 2, **dadurch gekennzeichnet,** **dass** die Abprodukte in den Prüfraum (12) abgegeben werden.

4. Verfahren nach einem der vorangehenden Ansprüche, **dadurch gekennzeichnet,** **dass** mittels einer Pumpe (19) der Brennstoffzellenanordnung (13) dem Kathodenraum in dem Prüfraum (12) befindliche Luft zugeführt wird.

5. Verfahren nach einem der vorangehenden Ansprüche, **dadurch gekennzeichnet,** **dass** mittels eines Brenngasdosierventils (17) der Brennstoffzellenanordnung (13) dem Anodenraum als ein Brenngas Wasserstoff ($H_2$) zugeführt wird.

6. Verfahren nach Anspruch 5, **dadurch gekennzeichnet,** **dass** der Wasserstoff ($H_2$) und der Sauerstoff ($O_2$) mittels eines in dem Prüfraum (12) befindlichen Elektrolyseurs hergestellt wird.

7. Verfahren nach einem der vorangehenden Ansprüche, **dadurch gekennzeichnet,** **dass** die Lufttemperatur in dem Prüfraum (12) mittels einer Temperiervorrichtung der Prüfkammer (10) eingestellt wird.

8. Verfahren nach einem der vorangehenden Ansprüche, **dadurch gekennzeichnet,** **dass** die relative Luftfeuchte in dem Prüfraum (12) mittels eines in dem Prüfraum angeordneten Befeuchters (29) und/oder eines Entfeuchters (28) der Prüfkammer (10) eingestellt wird.

9. Verfahren nach einem der vorangehenden Ansprüche, **dadurch gekennzeichnet,** **dass** mittels eines Feuchtesensors eines Befeuchterregelkreises der Steuervorrichtung die relative Luftfeuchte in dem Prüfraum (12) gemessen wird.

10. Verfahren nach einem der vorangehenden Ansprüche,

**dadurch gekennzeichnet,**

**dass** der Luftdruck in dem Prüfraum (12) mittels eines Zuluftgebläses und/oder eines Abluftgebläses der raumlufttechnischen Anlage (32) eingestellt wird.

11. Verfahren nach einem der vorangehenden Ansprüche,

**dadurch gekennzeichnet,**

**dass** mittels der raumlufttechnischen Anlage (32) eine Luftwechselrate in dem Prüfraum (12) und/oder eine Druckdifferenz zwischen dem Prüfraum und einer Umgebung (11) ausgebildet wird.

12. Verfahren nach einem der vorangehenden Ansprüche,

**dadurch gekennzeichnet,**

**dass** die Lufttemperatur und/oder die relative Luftfeuchte der Zuluft mittels der raumlufttechnischen Anlage (32) eingestellt wird.

13. Verfahren nach einem der vorangehenden Ansprüche,

**dadurch gekennzeichnet,**

**dass** ein mit der Brennstoffzellenanordnung (13) erzeugter elektrischer Strom gemessen wird, wobei die Steuervorrichtung die relative Luftfeuchte in Abhängigkeit des erzeugten Stroms regelt.

14. Verfahren nach einem der vorangehenden Ansprüche,

**dadurch gekennzeichnet,**

**dass** ein mit der Brennstoffzellenanordnung (13) erzeugter elektrischer Strom gemessen wird, wobei die Regeleinrichtung die Sauerstoffkonzentration in Abhängigkeit des erzeugten Stroms regelt.

15. Verfahren nach Anspruch 13 oder 14,

**dadurch gekennzeichnet,**

**dass** der gemessene elektrische Strom als eine Eingangsgröße eines Führungsreglers einer Kaskadenregelung der Steuervorrichtung verwendet wird.

16. Verfahren nach Anspruch 15,

**dadurch gekennzeichnet,**

**dass** die Regeleinrichtung als ein Folgeregler der Kaskadenregelung verwendet wird.

17. Verfahren nach einem der vorangehenden Ansprüche,

**dadurch gekennzeichnet,**

**dass** die physikalische Prüfbedingung eine Temperatur, eine relative Luftfeuchte, eine Korrosionsatmosphäre und/oder eine Bauteilfestigkeit ist.

18. Prüfkammer (10), insbesondere Klimakammer zur Konditionierung von Luft, umfassend einen gegenüber einer Umgebung (11) verschließbaren und temperaturisolierten Prüfraum (12) mit einer darin angeordneten Brennstoffzellenanordnung (13), die in dem Prüfraum betreibbar und zumindest einer physikalischen Prüfbedingung aussetzbar ist, wobei die Brennstoffzellenanordnung zumindest eine elektrochemische Brennstoffzelle (15) mit einem Anodenraum und einem Kathodenraum mit jeweils einer Zuführöffnung zur Zuführung von Reaktanten und einer Abführöffnung zur Abführung von Abprodukten der Brennstoffzellenanordnung aufweist, wobei der Brennstoffzellenanordnung als Reaktanten ein Brenngas und ein im Prüfraum vorhandenes Oxidationsgas zuführbar sind, wobei die Prüfkammer eine Steuervorrichtung zur Einstellung der Prüfbedingung durch eine Steuerung und/oder Regelung einer Lufttemperatur, eines Luftdrucks und einer relativen Luftfeuchte in dem Prüfraum aufweist, wobei die Prüfkammer eine raumlufttechnische Anlage (32) zur Zuführung konditionierter Zuluft in den Prüfraum und zur Abführung von Abluft aus dem Prüfraum aufweist, wobei die raumlufttechnische Anlage von der Steuervorrichtung steuerbar ist,

**dadurch gekennzeichnet,**

**dass** die Steuervorrichtung eine Regeleinrichtung mit einem Sensor (36) zur Bestimmung einer Sauerstoffkonzentration in dem Prüfraum aufweist, wobei die Regeleinrichtung zur Regelung der für einen Betrieb der Brennstoffzellenanordnung erforderlichen Sauerstoffkonzentration in dem Prüfraum ausgebildet ist.

19. Prüfkammer nach Anspruch 18,

**dadurch gekennzeichnet,**

**dass** die Prüfkammer (10) eine Temperiervorrichtung zur Temperierung des Prüfraums (12) aufweist, wobei mittels der Temperiervorrichtung eine Temperatur in einem Temperaturbereich von -70 °C bis +180 °C, vorzugsweise -80 °C bis +200 °C, innerhalb des Prüfraums ausbildbar ist, wobei die Temperiervorrichtung eine Kühleinrichtung mit einem Kühlkreislauf mit einem Kältemittel, einem Wärmeübertrager (30), der in dem Prüfraum angeordnet ist, einem Verdichter, einem Kondensator und einem Expansionsorgan aufweist, wobei die Temperiervorrichtung eine Heizeinrichtung mit einer Heizung und einem weiteren Wärmeübertrager (31) aufweist.

## Claims

1. A method for controlling a test chamber (10), in particular a climate test chamber for conditioning air, a fuel cell assembly (13) being exposed to at least one physical test condition in a test space (12) of the test chamber, the fuel cell assembly comprising at least one electrochemical fuel cell (15) having an anode compartment and a cathode compartment each having a feed opening for introducing reactants and a discharge opening for discharging waste products of the fuel cell assembly, the fuel cell assembly being operated in the test space, a fuel gas and an oxidation gas present in the test space being fed to the fuel cell assembly as reactants, the test condition being set by open-loop and/or closed-loop control of an air temperature, an air pressure and a relative humidity in the test space by means of a control device of the test chamber, the test space being supplied with conditioned supply air and exhaust air being discharged from the test space by means of an air conditioning and ventilation system (32) of the test chamber, the air conditioning and ventilation system being controlled by the control device,

**characterized in that**
an oxygen concentration is determined using a sensor (36) of a controller of the control device in the test space, the controller controlling the oxygen concentration required for operating the fuel cell arrangement.

2. The method according to claim 1,
**characterized in that**
oxygen ($O_2$) is introduced into the test space (12) and/or into a supply air duct (33) of the air conditioning and ventilation system (32) as a function of the oxygen concentration using a metering valve (21) of the controller.

3. The method according to claim 1 or 2,
**characterized in that**
the waste products are discharged into the test space (12).

4. The method according to any one of the preceding claims,
**characterized in that**
the cathode compartment is supplied with air present in the test space (12) by means of a pump (19) of the fuel cell assembly (13).

5. The method according to any one of the preceding claims,
**characterized in that**
the anode compartment is supplied with hydrogen ($H_2$) as a fuel gas by means of a fuel gas metering valve (17) of the fuel cell assembly (13).

6. The method according to claim 5,
**characterized in that**
the hydrogen ($H_2$) and the oxygen ($O_2$) are produced by means of an electrolyzer located in the test space (12).

7. The method according to any one of the preceding claims,
**characterized in that**
the air temperature in the test space (12) is set by means of a temperature control device of the test chamber (10).

8. The method according to any one of the preceding claims,
**characterized in that**
the relative humidity in the test space (12) is set by means of a humidifier (29) and/or a dehumidifier (28) of the test chamber (10), the humidifier (29) and/or the dehumidifier (28) being located in the test space.

9. The method according to any one of the preceding claims,
**characterized in that**
the relative humidity in the test space (12) is measured by means of a humidity sensor of a humidifier control circuit of the control device.

10. The method according to any one of the preceding claims,
**characterized in that**
the air pressure in the test space (12) is set by means of a supply air blower and/or an exhaust air blower of the air conditioning and ventilation system (32).

11. The method according to any one of the preceding claims,
**characterized in that**
the air conditioning and ventilation system (32) is used to establish an air exchange rate in the test space (12) and/or a pressure difference between the test space and an environment (11).

12. The method according to any one of the preceding claims,
**characterized in that**
the air temperature and/or the relative humidity of the supply air are set by means of the air conditioning and ventilation system (32).

13. The method according to any one of the preceding claims,
**characterized in that**
an electric current produced by the fuel cell assembly (13) is measured, the control device controlling the relative humidity as a function of the generated current.

14. The method according to any one of the preceding claims,
**characterized in that**
an electric current produced by the fuel cell assembly (13) is measured, the controller controlling the oxygen concentration as a function of the generated current.

15. The method according to claim 13 or 14,
**characterized in that**
the measured electric current is used as an input parameter of a master controller of a cascade control of the control device.

16. The method according to claim 15,
**characterized in that**
the controller is used as a slave controller of the cascade control.

17. The method according to any one of the preceding claims,
**characterized in that**
the physical test condition is a temperature, a relative humidity, a corrosive atmosphere and/or a component strength.

18. A test chamber (10), in particular a climate test chamber for conditioning air, comprising a test space (12) which can be sealed against an environment (11) and which is temperature-insulated, the test space (12) having disposed therein a fuel cell assembly (13) which is operable in the test space and exposable to at least one physical test condition, the fuel cell assembly comprising at least one electrochemical fuel cell (15) having an anode compartment and a cathode compartment each having a feed opening for introducing reactants and a discharge opening for discharging waste products of the fuel cell assembly, a fuel gas and an oxidation gas present in the test space being fed to the fuel cell assembly as reactants, the test chamber having a control device for setting the test condition by open-loop and/or closed-loop control of an air temperature, an air pressure and a relative humidity in the test space, the test chamber having an air conditioning and ventilation system (32) for introducing conditioned supply air into the test space and for discharging exhaust air from the test space, the air conditioning and ventilation system being controllable by the control device,
**characterized in that**
the control device has a controller having a sensor (36) for determining an oxygen concentration in the test space, the controller being configured to control the oxygen concentration in the test space required for operating the fuel cell arrangement.

19. The test chamber according to claim 18,
**characterized in that**
the test chamber (10) has a temperature control device for controlling the temperature of the test space (12), a temperature in a temperature range of -70 °C to +180 °C, preferably -80 °C to +200 °C, being establishable within the test space by means of the temperature control device, the temperature control device having a cooling unit comprising a cooling circuit comprising a refrigerant, a heat exchanger (30), which is disposed in the test space, a compressor, a condenser and an expansion element, the temperature control device having a heating unit comprising a heater and another heat exchanger (31).

**Revendications**

1. Procédé de commande d'une chambre d'essai (10), notamment d'une chambre d'essai climatique pour le conditionnement de l'air, un agencement (13) de cellules élémentaires à combustible étant exposé à au moins une condition d'essai physique dans un espace d'essai (12) de la chambre d'essai, l'agencement de cellules élémentaires à combustible comprenant au moins une cellule élémentaire à combustible (15) électrochimique ayant un compartiment anodique et un compartiment cathodique ayant chacun une ouverture d'alimentation pour introduire des réactifs et une ouverture d'évacuation pour évacuer les déchets de l'agencement de cellules élémentaires à combustible, l'agencement de cellules élémentaires à combustible étant en service dans l'espace d'essai, un gaz combustible et un gaz d'oxydation présents dans l'espace d'essai étant amenés à l'agencement de cellules élémentaires à combustible en tant que réactifs, la condition d'essai étant adaptée par commande en boucle ouverte et/ou en boucle fermée d'une température de l'air, d'une pression de l'air et d'une humidité relative dans l'espace d'essai au moyen d'un dispositif de contrôle de la chambre d'essai, l'espace d'essai étant alimenté en air frais conditionné et l'air vicié étant évacué de l'espace d'essai au moyen d'un système (32) de climatisation et de ventilation de la chambre d'essai, le système de climatisation et de ventilation étant commandé par le dispositif de contrôle,
**caractérisé en ce**
**qu'**une concentration en oxygène est déterminée à l'aide d'un capteur (36) d'un régulateur du dispositif de contrôle dans l'espace d'essai, le régulateur contrôlant la concentration en oxygène requise pour faire fonctionner l'agencement de cellule élémentaire à combustible.

2. Procédé selon la revendication 1,
**caractérisé en ce que**
de l'oxygène ($O_2$) est introduit dans l'espace d'essai (12) et/ou dans un conduit (33) de l'air frais du système (32) de climatisation et de ventilation en fonction de la concentration en oxygène à l'aide d'un doseur (21) du régulateur.

3. Procédé selon la revendication 1 ou la revendication 2,
**caractérisé en ce que**
les déchets sont évacués dans l'espace d'essai (12).

4. Procédé selon l'une quelconque des revendications précédentes, **caractérisé en ce que**
le compartiment cathodique est alimenté en air présent dans l'espace d'essai (12) au moyen d'une pompe (19) de l'agencement (13) de cellules élémentaires à combustible.

5. Procédé selon l'une quelconque des revendications précédentes, **caractérisé en ce que**
le compartiment anodique est alimenté en hydrogène ($H_2$) en tant que gaz combustible au moyen d'un doseur de gaz combustible (17) de l'agencement (13) de cellules élémentaires à combustible.

6. Procédé selon la revendication 5,
**caractérisé en ce que**
l'hydrogène ($H_2$) et l'oxygène ($O_2$) sont produits au moyen d'un électrolyseur situé dans l'espace d'essai (12).

7. Procédé selon l'une quelconque des revendications précédentes, **caractérisé en ce que**
la température de l'air dans l'espace d'essai (12) est adaptée au moyen d'un dispositif de contrôle de la température de la chambre d'essai (10).

8. Procédé selon l'une quelconque des revendications précédentes, **caractérisé en ce que**
l'humidité relative dans l'espace d'essai (12) est adaptée au moyen d'un humidificateur (29) et/ou d'un déshumidificateur (28) de la chambre d'essai (10), l'humidificateur (29) et/ou le déshumidificateur (28) étant situé dans l'espace d'essai.

9. Procédé selon l'une quelconque des revendications précédentes, **caractérisé en ce que**
l'humidité relative dans l'espace d'essai (12) est mesurée au moyen d'un capteur d'humidité d'un circuit de commande de l'humidificateur du dispositif de contrôle.

10. Procédé selon l'une quelconque des revendications précédentes, **caractérisé en ce que**
la pression de l'air dans l'espace d'essai (12) est adaptée au moyen d'un ventilateur de l'air frais et/ou d'un ventilateur de l'air vicié du système (32) de climatisation et de ventilation.

11. Procédé selon l'une quelconque des revendications précédentes, **caractérisé en ce que**
le système (32) de climatisation et de ventilation est utilisé pour établir un taux de renouvellement de l'air dans l'espace d'essai (12) et/ou une différence de pression entre l'espace d'essai et un environnement (11).

12. Procédé selon l'une quelconque des revendications précédentes, **caractérisé en ce que**
la température de l'air et/ou l'humidité relative de l'air frais sont adaptées au moyen du système (32) de climatisation et de ventilation.

13. Procédé selon l'une quelconque des revendications précédentes, **caractérisé en ce qu'**un courant électrique produit par l'agencement (13) de cellules élémentaires à combustible est mesuré, le dispositif de contrôle contrôlant l'humidité relative en fonction du courant généré.

14. Procédé selon l'une quelconque des revendications précédentes, **caractérisé en ce qu'**un courant électrique produit par l'agencement (13) de cellules élémentaires à combustible est mesuré, le régulateur contrôlant la concentration en oxygène en fonction du courant généré.

15. Procédé selon la revendication 13 ou la revendication 14, **caractérisé en ce que**
le courant électrique mesuré est utilisé comme grandeur entrée d'un régulateur principal d'une régulation en cascade du dispositif de contrôle.

16. Procédé selon la revendication 15,
**caractérisé en ce que**
le régulateur est utilisé comme régulateur en esclave de la régulation en cascade.

17. Procédé selon l'une quelconque des revendications précédentes, **caractérisé en ce que**
la condition d'essai physique est une température, une humidité relative, une atmosphère corrosive et/ou une rigidité des composants.

18. Chambre d'essai (10), notamment une chambre d'essai climatique pour le conditionnement de l'air, comprenant un espace d'essai (12) qui peut être isolé de l'environnement (11) et qui est isolé thermiquement, l'espace d'essai (12) ayant un agencement (13) de cellules élémentaires à combustible qui est y disposé et qui peut être en service dans l'espace d'essai et être exposé à au moins une condition d'essai physique, l'agencement de cellules élémentaires à combustible comprenant au moins une cellule élémentaire à combustible (15) électrochimique ayant un compartiment anodique et un compartiment cathodique ayant chacun une ouverture d'alimentation pour introduire des réactifs et une ouverture d'évacuation pour évacuer les déchets de l'agencement de cellules élémentaires à combustible, un gaz combustible et un gaz d'oxydation présents dans l'espace d'essai étant amenés à l'agencement de cellules élémentaires à combustible en tant que réactifs, la chambre d'essai ayant un dispositif de contrôle pour adapter la condition d'essai par commande en boucle ouverte et/ou en boucle fermée d'une température de l'air, d'une pression de l'air et d'une humidité relative dans l'espace d'essai, la chambre d'essai ayant un système (32) de climatisation et de ventilation pour amener de l'air frais conditionné dans l'espace d'essai et pour évacuer de l'air vicié de l'espace d'essai, le système de climatisation et de ventilation pouvant être commandé par le dispositif de contrôle,
**caractérisée en ce que**
le dispositif de contrôle a un régulateur ayant un capteur (36) pour déterminer une concentration en oxygène dans l'espace d'essai, le régulateur étant configuré pour contrôler la concentration en oxygène dans l'espace d'essai requise pour faire fonctionner l'agencement de cellule élémentaire à combustible.

19. Chambre d'essai selon la revendication 18,
**caractérisée en ce que**
la chambre d'essai (10) a un dispositif de contrôle de la température de l'espace d'essai (12), une température comprise dans une plage de température de -70 °C à +180 °C, de préférence de -80 °C à +200 °C, pouvant être établie dans l'espace d'essai au moyen du dispositif de contrôle de la température, le dispositif de contrôle de la température a une unité de refroidissement comprenant un circuit de refroidissement comprenant un réfrigérant, un échangeur de chaleur (30) qui est disposé dans l'espace d'essai, un compresseur, un condenseur et un élément d'expansion, le dispositif de contrôle de la température a une unité de chauffage comprenant un chauffage et un autre échangeur de chaleur (31).

Figur

EP 3 650 832 B1

**EP 3 650 832 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 20013299 U1